(19) Europäisches Patentamt
European Patent Office
Office européen des brevets

(11) Veröffentlichungsnummer: **0 411 676 B1**

(12) # EUROPÄISCHE PATENTSCHRIFT

(45) Veröffentlichungstag der Patentschrift: **15.06.94**

(51) Int. Cl.[5]: **C07D 319/12**, C07D 317/14, C07D 317/22, A01N 43/28, A01N 43/30

(21) Anmeldenummer: **90117463.1**

(22) Anmeldetag: **13.01.88**

(60) Veröffentlichungsnummer der früheren Anmeldung nach Art. 76 EPÜ: **0 276 196**

(54) **Substituierte Dioxanderivate.**

(30) Priorität: **19.01.87 CH 177/87**

(43) Veröffentlichungstag der Anmeldung: **06.02.91 Patentblatt 91/06**

(45) Bekanntmachung des Hinweises auf die Patenterteilung: **15.06.94 Patentblatt 94/24**

(84) Benannte Vertragsstaaten: **AT BE CH DE ES FR GB GR IT LI NL**

(56) Entgegenhaltungen:
**US-A- 4 007 280**
**US-A- 4 097 581**
**US-A- 4 590 282**

(73) Patentinhaber: **CIBA-GEIGY AG**
**Klybeckstrasse 141**
**CH-4002 Basel(CH)**

(72) Erfinder: **Karrer, Friedrich. Dr.**
**Rebbergstrasse 5**
**CH-4800 Zofingen(CH)**

EP 0 411 676 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neuartige substituierte 1,4-Dioxan-derivate, Verfahren zu ihrer Herstellung und ihre Verwendung in der Schädlingsbekämpfung.

Die erfindungsgemässen Verbindungen haben die Formel I

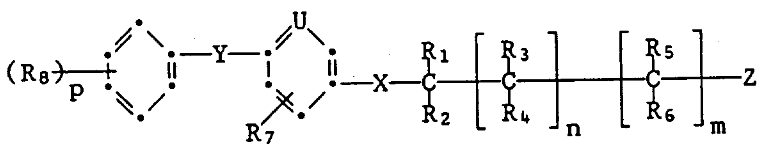

(I),

worin

| | |
|---|---|
| $R_1$, $R_2$, $R_3$, $R_4$, $R_5$ und $R_6$ | unabhängig voneinander Wasserstoff oder $C_1$-$C_4$-Alkyl oder, wenn n = 1, |
| $R_2$ und $R_4$ | zusammen einen der Reste $-(CH_2)_3-$ oder $-(CH_2)_4-$ ; |
| $R_7$ | Wasserstoff, Halogen, Methyl, Aethyl, mit 1 bis 5 Halogenatomen substituiertes $C_1$-$C_2$-Alkyl, Methoxy, Aethoxy oder mit 1 bis 5 Halogenatomen substituiertes $C_1$-$C_2$-Alkoxy; |
| $R_8$ | Halogen, $C_1$-$C_3$-Alkyl, mit 1 bis 7 Halogenatomen substituiertes $C_1$-$C_3$-Alkyl, $C_1$-$C_3$-Alkoxy, mit 1 bis 7 Halogenatomen substituiertes $C_1$-$C_3$-Alkoxy oder Cyano; |
| U | eine Gruppierung -CH= oder -N= ; |
| X | -O- , -S- oder -N($R_9$)- ; |
| Y | -O- , -S- , -S(O)- , -S($O_2$)- , -$CH_2$- , -CO- oder -N($R_9$)- , wobei |
| $R_9$ | für Wasserstoff, $C_1$-$C_4$-Alkyl oder Alkylcarbonyl mit insgesamt 2 bis 4 C-Atomen steht; |
| n | eine Zahl 0 oder 1; |
| m | eine Zahl 0, 1 oder 2; |
| p | eine Zahl 1, 2 oder 3; und |
| Z | den Rest |

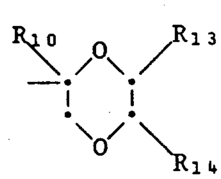

bedeuten, wobei

| | |
|---|---|
| $R_{10}$ | Wasserstoff, Methyl oder Aethyl; und |
| $R_{13}$ und $R_{14}$ | unabhängig voneinander Wasserstoff, Halogen oder $C_1$-$C_3$-Alkyl; oder |
| $R_{13}$ und $R_{14}$ | zusammen die Gruppe |

$$-(CH_2)_4-$$

bedeuten.

Halogen steht im Rahmen der vorliegenden Erfindung für Fluor, Chlor, Brom oder Jod, vorzugsweise für Fluor oder Chlor.

Der Erfindungsgegenstand umfasst auch die möglichen Isomeren, einschliesslich der Diastereomeren und der Enantiomeren, der Verbindungen der Formel I.

Die Alkyl-, Alkoxy-, Halogenalkyl-, Halogenalkoxy-, Alkoxyalkyl, Alkylthioalkyl-, Alkenyl- und Alkinylgruppen können geradkettig oder verzweigt sein. Beispiele solcher Gruppen sind u.a. Methyl, Methoxy, Methoxymethyl, Difluormethoxy, Aethyl, Aethoxy, 2-Fluoräthoxy, 1,1,2,2-Tetrafluoräthyl, Pentafluoräthyl, 1,1,2,2-Tetrafluoräthoxy, Pentafluoräthoxy, Propyl, Isopropyl, n-Butyl, n-Pentyl, n-Hexyl und deren Isomere, Vinyl, 1-Propen-3-yl, 1-Propinyl.

Hervorzuheben sind auch Verbindungen der Formel I, die dadurch gekennzeichnet sind, dass

$R_1$ und $R_3$ unabhängig voneinander Wasserstoff, Methyl oder Aethyl;
$R_2$, $R_4$, $R_5$ und $R_6$ Wasserstoff oder, wenn n = 1,
$R_2$ und $R_4$ zusammen einen der Reste

$$-(CH_2)_3- \text{ oder } -(CH_2)_4- ;$$

$R_7$ Wasserstoff oder Halogen;
$R_8$ Methyl, Aethyl, Halogen oder mit 1 bis 5 Halogenatomen substituiertes $C_1$-$C_2$-Alkyl;
U eine Gruppierung -CH= oder -N= ;
X -O- , -S- oder -N($R_9$)- ;
Y -O- , -S- , -$CH_2$- oder -CO- ; wobei
$R_9$ für Wasserstoff, Methyl oder Acetyl steht;
n und m unabhängig voneinander eine Zahl O oder 1; und
p eine Zahl 1 oder 2; und
Z den Rest

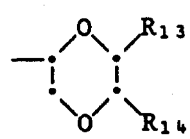

bedeuten; wobei
$R_{13}$ und $R_{14}$ unabhängig voneinander Wasserstoff oder Methyl bedeuten.

Weiterhin bevorzugt sind diejenigen Verbindungen der Formel I, worin
$R_1$ Wasserstoff oder Methyl;
$R_2$, $R_3$, $R_4$, $R_5$, $R_6$ und $R_7$ Wasserstoff;
$R_8$ Fluor, Chlor, Methyl oder Trifluormethyl;
U die Gruppierung -CH= ;
X -O- ;
Y -O- ;
n und m die Zahl 0;
p eine Zahl 1 oder 2; und
Z den Rest

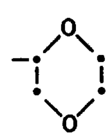

bedeuten;
diejenigen Verbindungen der Formel I, worin
$R_1$ Wasserstoff oder Methyl;
$R_2$, $R_3$, $R_4$, $R_5$, $R_6$ und $R_7$ Wasserstoff
$R_8$ Halogen oder Trifluormethyl;
U die Gruppierung -CH=;
X -O- oder -S-;
Y -O- , -S- , -$CH_2$- oder -CO-;
n und m die Zahl 0;

| | |
|---|---|
| p | eine Zahl 1 oder 2; und |
| Z | den Rest |

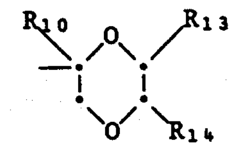

bedeuten, wobei

| | |
|---|---|
| $R_{10}$ | Wasserstoff oder Methyl; und |
| $R_{13}$ und $R_{14}$ | unabhängig voneinander Wasserstoff, Halogen oder $C_1$-$C_3$-Alkyl; oder |
| $R_{13}$ und $R_{14}$ | zusammen die Gruppe |

$$-(CH_2)_4-$$

bedeuten; und

diejenigen Verbindungen der Formel I, worin

| | |
|---|---|
| $R_1$, $R_2$, $R_3$, $R_4$, $R_5$, $R_6$ und $R_7$ | Wasserstoff; |
| $R_8$ | Fluor, Chlor oder Trifluormethyl; |
| U | die Gruppierung -CH=; |
| X | -O- , |
| Y | -O- , -S- , $-CH_2-$ oder -CO- ; |
| n und m | die Zahl 0; |
| p | eine Zahl 1 oder 2; und |
| Z | den Rest |

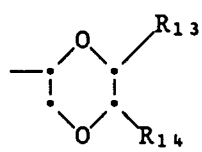

bedeuten, wobei

| | |
|---|---|
| $R_{13}$ und $R_{14}$ | unabhängig voneinander Wasserstoff, Halogen oder $C_1$-$C_3$-Alkyl; oder |
| $R_{13}$ und $R_{14}$ | zusammen die Gruppe |

$$-(CH_2)_4-$$

bedeuten.

Besondere Bedeutung kommt denjenigen Verbindungen der Formel I zu, in denen p = 1 ist.

Die Verbindungen der Formel I können in an sich bekannter Weise hergestellt werden (vgl. auch US-Patentschrift No. 4,007,280):

Man kann hierzu z.B. eine Verbindung der Formel II

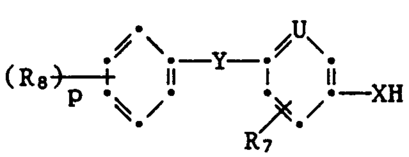

(II)

mit einer Verbindung der Formel III

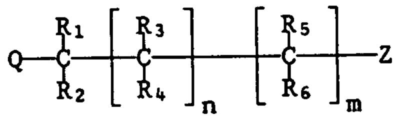

(III)

umsetzen, wobei in den Formeln II und III $R_1$ bis $R_8$, U, X, Y, n, m, p und Z die vorstehend angegebenen Bedeutungen haben und Q für eine Abgangsgruppe steht.

Die Durchführung des obigen Verfahrens erfolgt vorzugsweise in Gegenwart eines gegenüber den Umsetzungsteilnehmern inerten Lösungs- oder Verdünnungsmittels und mindestens eines Aequivalents eines Säureacceptors bzw. einer basischen Substanz. Als Säureacceptoren, bzw. Basen kommen insbesondere tertiäre Amine, wie Trialkylamine und Pyridin, ferner Hydride, Hydroxide, Oxide, Carbonate und Hydrogencarbonate von Alkali- und Erdalkalimetallen sowie auch Alkalimetallalkoholate, wie z.B. Kalium-t-butylat und Natriummethylat usw., in Betracht. Die Reaktions-temperatur liegt je nach der Art der verwendeten Lösungsmittel üblicherweise im Bereich von -5 °C bis +140 °C, vorzugsweise zwischen 0 und 60 °C. Bei der Ausgangsverbindung der Formel III steht Q für eine herkömmliche Abgangsgruppe, wie z.B. ein Halogenatom die Mesyloxy- oder Tosyloxy-Gruppe.

Das vorstehend aufgeführte Verfahren wird bevorzugt bei normalem oder erhöhtem Druck und vorzugsweise in einem inerten Lösungs- oder Verdünnungsmittel durchgeführt. Als Lösungs- oder Verdünnungsmittel für Verfahren eignen sich z.B. Aether und ätherartige Verbindungen, wie Diäthyläther, Diisopropyläther, Dioxan, 1,2-Dimethoxyäthan und Tetrahydrofuran; Amide, wie N,N-dialkylierte Carbonsäureamide; Sulfolan und Dimethylsulfoxid.

Die Ausgangsstoffe der Formeln II und III sind bekannt oder können, falls sie neu sind, analog bekannten Methoden hergestellt werden. Sie bilden dann ebenfalls einen Gegenstand der vorliegenden Erfindung.

Die Verbindungen der Formel I können als Gemische der verschiedenen enantiomeren Formen vorliegen, bzw. bei der Synthese gebildet werden. Die Diastereomerengemische bzw. Racemate der Formel I können nach bekannten Methoden in die einzelnen Formen aufgetrennt werden. Unter der Verbindung der Formel I versteht man daher sinngemäss sowohl die einzelnen diastereoisomeren resp. enantiomeren Formen, als auch deren Gemische.

Aus der US-PS 4.097.581 sind bereits 4-(4-Phenoxy)-phenoxymethyl-1,3-dioxolane als Insektizide bekannt. Aehnlich strukturierte 1,3-Dioxolan-und 1,4-Benzodioxin-Derivate und deren Anwendung als Pestizide sind Gegenstand von US-PS 4.590.282. In der US-PS 4.007.280 werden pestizid wirksame 2-(4-Phenoxy)-phenoxymethyl-1,4-benzodioxane beschrieben. Demgegenüber unterscheiden sich die erfindungsgemässen Verbindungen der Formel I im wesentlichen durch das Vorliegen einer obligat mit 1 bis 3 Resten $R_8$ substituierten Phenylgruppe und einer 1, 4-Dioxan-2-yl-Gruppe ohne anneliertes Benzolsystem.

Es wurde überraschenderweise gefunden, dass sich die erfindungsgemässen Verbindungen der Formel I ausgezeichnet zur Bekämpfung von verschiedenartigen Schädlingen an Tieren und Pflanzen sowie im Boden eignen. So können sie zur Bekämpfung von Insekten, z.B. der Ordnung Lepidoptera, Coleoptera, Homoptera, Heteroptera, Diptera, Thysano-ptera, Orthoptera, Anoplura, Siphonaptera, Mallophaga, Thysanura, Isoptera, Psocoptera und Hymenoptera und von Milben und Zecken der Ordnung Acarina eingesetzt werden.

So eignen sich Verbindungen der Formel I zur Bekämpfung von pflanzenschädigenden Insekten in Zier- und Nutzpflanzungen, insbesondere in Baumwollkulturen (z.B. Spodoptera littoralis und Heliothis virescens). Die Verbindungen der Formel I haben ferner eine günstige Wirkung gegen - Bodeninsekten (z.B. Aulacophora femoralis, Chortophila brassicae, Diabrotica balteata, Pachnoda savignyi und Scotia ypsilon) und insbesondere gegen saugende Insekten, wie Aphide (z.B. Myzus persicae, Aphis craccivora und Aonidiella aurantii sowie weitere Schildlausarten). Die erfindungsgemässen Verbindungen sind auch wirksam als Ovizide im Pflanzenschutz, speziell zur Bekämpfung von pflanzenschädigenden Insekten, wie z.B. Cydia pomonella, Lobesia botrana und Adoxophyes reticulana.

Wirkstoffe der Formel I zeigen auch eine sehr günstige Wirkung gegen Fliegen, wie z.B. Musca domestica und Mückenlarven sowie gegen Vorratsschädlinge, wie z.B. Sitophilus orizae und Rhizopertha dominica.

Die erfindungsgemässen Verbindungen der Formeln I weisen eine besonders ausgeprägte Wirkung gegen pflanzenschädigende Akariden (Spinnmilben: z.B. der Familien Tetranychidae, Tarsonemidae, Eriophydae, Tyroglyphidae und Glycyphagidae) und auch gegen ektoparasitäre Akariden (Milben und Zecken:

z.B. der Familien Ixodidae, Argasidae, Sacroptidae und Dermanyssidae), welche Nutztiere befallen, auf. Einige der erfindungsgemässen Verbindungen zeichnen sich durch gute akarizid-ovizide Aktivität und Blattpenetrationswirkung aus. Die erfindungsgemässen Verbindungen eignen sich vor allem zur Bekämpfung der folgenden, Obst-und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsomemus pallidus, Phyllocoptes vitis und Phyllocoptura oleivora.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org. Phosphorverbindungen; Nitrophenole und deren Derivate; Formamidine; Harnstoffe; pyrethrinartige Verbindungen sowie Karbamate und chlorierte Kohlenwasserstoffe.

Die gute pestizide Wirkung der erfindungsgemässen Verbindungen der Formeln I entspricht einer Abtötungsrate (Mortalität) von mindestens 50-60 % der erwähnten Schädlinge.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnis-sen entsprechend gewählt.

Die Formulierungen, d.h. die mindenstens einen Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctyl-phthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyl-äther, Essigester, Propylmyristat oder Propylpalmitat, Ketone wie Cyclohexanon, stark polare Lösungsmittel wie N-Methyl-2-pyrrolidon` Dimethylsulfoxid oder Dimethylformamid, sowie gegebenenfalls epoxidierte Pflanzenöle wie epoxidiertes Kokosnussöl oder Sojaöl; Silikonöle oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes und Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5-Aethylenglykoleinheiten.

Als Beispiele nichtionischer Tenside seien Nonylphenolpolyäthoxyäthanole, Ricinusölpolyglycoläther, Ricinusölthioxilat, Polypropylen-Polyäthylenoxid-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgenden Publikationen beschrieben:

"McCutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides, wobei sich die %-Angaben auf das Gewicht beziehen.

Während als Handelsware eher konzentrierte Mittel bevorzugt werden, verwendet der Endverbraucher in der Regel verdünnte Mittel bzw. Zubereitungen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1:

a) Herstellung von 2-Methansulfonylmethyl-6-methyl-1,4-dioxan (Ausgangsprodukt):

Zu einer Lösung von 21,1 g 2-Hydroxymethyl-6-methyl-1,4-dioxan, 15,2 g Pyridin und 0,6 g 4-Dimethylaminopyridin in 80 ml Dichlormethan tropft man unter Rühren bei 0 bis 5 °C während ca. 30 Minuten 13,7 ml Methansulfonsäurechlorid. Nach weiterem 18stündigem Rühren bei Raumtemperatur wird das Reaktionsgemisch wiederholt mit 1N-Salzsäure extrahiert und dann mit Wasser neutral gewaschen. Nach dem Trocknen der abgetrennten organischen Phase über Natriumsulfat und vollständigem Abdestillieren des Lösungsmittels erhält man die Titelverbindung der Formel

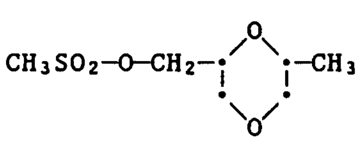

Das $^1H$-Protonenresonanzspektrum steht mit dieser Struktur in Einklang; $n_D^{22}$ = 1,4607.

b) Herstellung von 2-[4-(3-Fluorphenoxy)]-phenoxymethyl-6-methyl-1,4-dioxan:

Zu einer Lösung von 6,2 g 4-(3-Fluorphenoxy)-phenol in 20 ml Dimethylsulfoxid gibt man unter Eiskühlung eine frisch bereitete Lösung von 3,6 g Kalium-tert.-butoxid in 20 ml Dimethylsulfoxid. Anschliessend wird zu der so erhaltenen Lösung des Kaliumsalzes dieses Phenols eine Lösung von 8 g des wie oben nach a) hergestellten 2-Methansulfonylmethyl-6-methyl-1,4-dioxans in 20 ml Dimethylsulfoxid bei

10°C während ca. 30 Minunten hinzugetropft. Das Reaktionsgemisch wird dann während weiteren 16 Stunden bei 22°C gerührt. Danach wird das Gemisch auf Eiswasser gegossen und wiederholt mit Diäthyläther-Hexan (1:1) extrahiert. Die vereinigten organischen Phasen werden mit 10%iger Kalilauge und schliesslich mit Wasser neutral gewaschen, über Natriumsulfat getrocknet und die Lösungsmittel abdestilliert. Durch Chromatographie an Kieselgel (Eluierungsmittel: Hexan-Essigsäureäthylester/6:1) erhält man die Titelverbindung der Formel

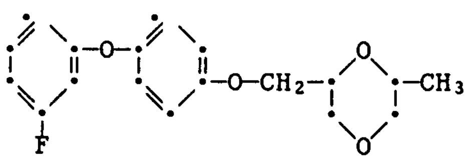

mit einem Brechungsindex $n_D^{22}$ = 1,5445 (Verbindung Nr. 1). Die Elementaranalyse und das $^1$H-NMR-Spektrum stehen mit der oben angegebenen Struktur in Einklang.

Analog der vorstehend beschriebenen Arbeitsweisen werden folgende Verbindungen der Formel I hergestellt:

| Verbindung Nr. | | physik. Daten |
|---|---|---|
| 2 | 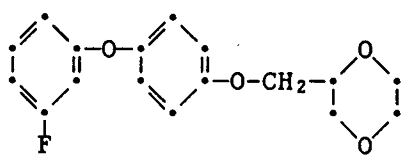 | Smp. 57-58°C |
| 3 | 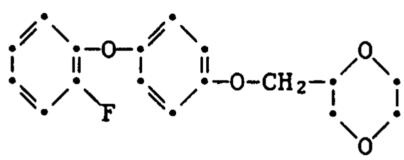 | Smp. 60-62°C |
| 4 | 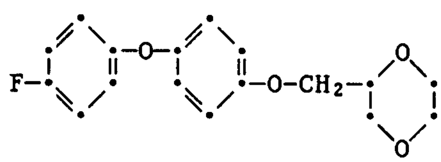 | Smp: 65-67°C |
| 5 | 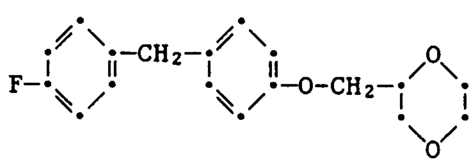 | Smp.66-67°C |
| 6 | 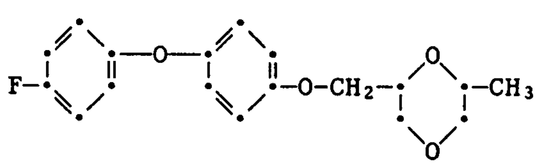 | Smp.~35°C |

Auf analoge Weise wie vorstehend angegeben kann auch die Verbindung der folgenden Formel hergestellt werden:

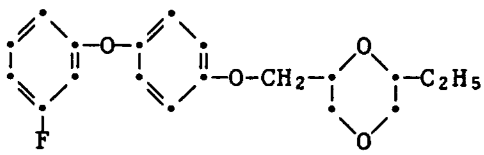

.

Beispiel 2:

Formulierungen für flüssige Wirkstoffe der Formel I gemäss Beispiel 1 (% = Gewichtsprozent)

| 1. Emulsions-Konzentrate | a) | b) | c) |
|---|---|---|---|
| Wirkstoff gemäss Herstellungsbeispielen | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |
| Aus solchen Konzentraten können durch Verdünnen mit Wasser Emulsionen jeder gewünschten Konzentration hergestellt werden. | | | |

| 2. Lösungen | a) | b) | c) | d) |
|---|---|---|---|---|
| Wirkstoff gemäss Herstellungsbeispielen | 80 % | 10 % | 5 % | 95 % |
| Aethylenglykol-monomethyläther | 20 % | - | - | - |
| Polyäthylenglykol (MG 400) | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190 °C) | - | - | 94 % | - |
| Die Lösungen sind zur Anwendung in Form kleinster Tropfen geeignet. | | | | |

| 3. Granulate | a) | b) |
|---|---|---|
| Wirkstoff gemäss Herstellungsbeispielen | 5 % | 10 % |
| Kaolin | 94 % | - |
| Hochdisperse Kieselsäure | 1 % | - |
| Attapulgit | - | 90 % |
| Der Wirkstoff wird in Methylenchlorid gelöst, auf den Träger aufgesprüht und das Lösungsmittel anschliessend im Vakuum abgedampft. | | |

| 4. Stäubemittel | a) | b) |
|---|---|---|
| Wirkstoff gemäss Herstellungsbeispielen | 2 % | 5 % |
| Hochdisperse Kieselsäure | 1 % | 5 % |
| Talkum | 97 % | - |
| Kaolin | - | 90 % |
| Durch inniges Vermischen der Trägerstoffe mit dem Wirkstoff erhält man gebrauchsfertige Stäubemittel. | | |

Formulierungen für feste Wirkstoffe der Formel I gemäss Beispiel 1 (% = Gewichtsprozent)

| 5. Spritzpulver | a) | b) | c) |
|---|---|---|---|
| Wirkstoff gemäss Herstellungsbeispielen | 20 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 67 % | 27 % | - |
| Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen. | | | |

| 6. Emulsions-Konzentrat | a) | b) |
|---|---|---|
| Wirkstoff gemäss Herstellungsbeispielen | 10 % | 10 % |
| Octylphenolpolyäthylenglykoläther (4-5 Mol AeO) | 3 % | - |
| Ca-Dodecylbenzolsulfonat | 3% | - |
| Ricinusölpolyglykoläther (36 Mol AeO) | 4 % | - |
| Ricinusölthioxilat | - | 25 % |
| Cyclohexanon | 30 % | - |
| Butanol | - | 15 % |
| Xylolgemisch | 50 % | - |
| Essigester | - | 50 % |
| Aus diesem Konzentrat können durch Verdünnen mit Wasser Emulsionen jeder gewünschten Konzentration hergestellt werden. | | |

| 7. Stäubemittel | a) | b) |
|---|---|---|
| Wirkstoff gemäss Herstellungsbeispielen | 5 % | 8 % |
| Talkum | 95 % | - |
| Kaolin | - | 92 % |
| Man erhält anwendungsfertige Stäubemittel, indem der Wirkstoff mit dem Träger vermischt und auf einer geeigneten Mühle vermahlen wird. | | |

| 8. Extruder Granulat | |
|---|---|
| Wirkstoff gemäss Herstellungsbeispielen | 10 % |
| Na-Ligninsulfonat | 2 % |
| Carboxymethylcellulose | 1 % |
| Kaolin | 87 % |
| Der Wirkstoff wird mit den Zusatzstoffen vermischt, vermahlen und mit Wasser angefeuchtet. Dieses Gemisch wird extrudiert und anschliessend im Luftstrom getrocknet. | |

| 9. Umhüllungs-Granulat | |
|---|---|
| Wirkstoff gemäss Herstellungsbeispielen | 3 % |
| Polyäthylenglykol (MG 200) | 3 % |
| Kaolin | 94 % |
| Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate. | |

| 10. Suspensions-Konzentrat | |
|---|---|
| Wirkstoff gemäss Herstellungsbeispielen | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |
| Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können. | |

Beispiel 3: Wirkung gegen Lucilia sericata

Zu 9 ml eines Zuchtmediums wird bei 50 °C ein ml einer 0,1 % Aktivsubstanz enthaltenden wässrigen Zubereitung hinzugefügt. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt. Verbindungen der Formel I gemäss Beispiel 1 zeigen in diesem Test gute Wirkung gegen Lucilia sericata.

Beispiel 4: Wirkung gegen Aëdes aegypti

Auf die Oberfläche von 150 ml Wasser, das sich in einem Behälter befindet, wird so viel einer 0,1%igen acetonischen Lösung des Wirkstoffs pipettiert, dass eine Konzentration von 100 ppm erhalten wird. Nach Verdunsten des Acetons wird der Behälter mit 30 bis 40 2-tägigen Aëdes-Larven beschickt. Nach 2 und 7 Tagen wird die % Mortalität (Anzahl der nicht mehr schwimmfähigen Larven) geprüft.

Verbindungen der Formel I gemäss Beispiel 1 zeigen gute Wirkung (Mortalität) im obigen Test.

Beispiel 5: Wirkung gegen Aonidiella aurantii

a) Tauchtest - Kartoffelknollen

Kleine Kartoffelknollen werden mit Wanderlarven ("crawlern") von Aonidiella aurantii (rote Citrus-Schildlaus) in der Weise infestiert, dass man die Kartoffeln während etwa 24 Stunden in direktem Kontakt mit stark von Aonodiella befallenen grossen Kürbissen hält, so dass schliesslich jede Kartoffel mit 200 bis 300 "crawlern" besiedelt wird. Wenn die Schildlaus Population auf den Kartoffeln das 2. Nymphenstadium erreicht hat, d.h. nach etwa 14 Tagen, werden die Kartoffeln für etwa 2 bis 3 Minuten mittels einer Zange in eine wässrige Emulsionszubereitung eingetaucht, die den Wirkstoff in einer Konzentration von 50 ppm enthält. Nach dem Trocknen der so behandelten Kartoffelknollen werden diese während 10 bis 12 Wochen in Plastikbehälter gebracht, die im oberen Teil mit einem Leimring versehen sind, welcher zum Abfangen der geflügelten Männchen dient. Danach wird zur Auswertung der Zustand der behandelten Schildlaus-Population mit demjenigen von unbehandelten Kontrollansätzen verglichen, wobei im einzelnen auf die Anzahl der Männchen, Entwicklung der Weibchen (Ausbildung von Schilden) und Produktion von "crawlern" der ersten Folgegeneration bonitiert wird.

b) Sprühbehandlung - Citruspflanzen

Stecklinge von Citrus trifoliata werden mit einem stark von Aonodiella befallenen Kürbis (vgl. vorstehend Test a) in engen Kontakt gebracht, so dass jeder Steckling mit etwa 150 bis 200 übergewanderten "crawlern" besiedelt wird. Wenn die Schildlaus-Population auf den Stecklingen das 2. Nymphenstadium erreicht hat, d.h. nach etwa 14 Tagen, werden die infestierten Stecklinge bis zur Tropfnässe mit einer wässrigen Emulsionszubereitung besprüht, die 50 ppm des zu prüfenden Wirkstoffes enthält. Nach etwa 10 bis 12 Wochen wird auf die Entwicklung der Population (%-Anteil männliche und weibliche Schilde) und Produktion von "crawlern" gegenüber unbehandelten Kontrollen bonitiert.

Verbindungen Nr. 1 bis 4 gemäss Beispiel 1 zeigen 100 % Wirkung in diesen Testen.

Beispiel 6: Insektizide Frassgift-Wirkung

Ca. 25 cm hohe eingetopfte Baumwollpflanzen werden mit einer wässrigen Wirkstoffemulsion besprüht, die den jeweiligen Wirkstoff in einer Konzentration von 400 ppm enthält.

Nach dem Antrocknen des Sprühbelages werden pro Konzentration je 3 Baumwollpflanzen in einem Metallkessel gestellt und mit 50 Spodoptera littoralis-Larven im ersten larvalen Stadium besiedelt. Der Kessel wird dann mit einer Glasplatte abgedeckt. Der Versuch wird bei 28 °C und etwa 60 % relativer Luftfeuchtigkeit durchgeführt. Nach 96 Stunden wird die %-Mortalität der Test-Insekten gegenüber unbehandelten Kontrollen bestimmt.

Verbindungen der Formel I gemäss Beispiel 1 zeigen gute Wirkung in diesem Test.

Beispiel 7: Wirkung gegen Zecken

Als Testtiere werden mit Blut vollgesogene adulte Weichen der Rinderzecke Boophilus microplus verwendet. Es werden je 10 Zecken eines OP-resistenten Stammes (z.B. vom Biarra-Stamm) und eines normal empfindlichen Stammes (z.B. vom Yeerongpilly-Stamm) behandelt. Die Zecken werden auf mit Doppelklebeband bezogenen Platten dorsal fixiert und dann mit einem mit wässrigen Emulsionen bzw. Lösungen enthaltend 400 ppm der zu untersuchenden Verbindung getränkten Wattebausch während 1 Stunde bedeckt. Nach Entfernung des Wattebausches werden die Zecken über Nach bei 24 °C getrocknet und anschliessend in einem klimatisierten Raum bei konstanten Bedingungen (28 °C, 80 % rel. Luftfeuchtigkeit) gehalten, und zwar für 4 Wochen bis zum Ende der Eiablage und Beginn des Larvenschlupfes. Zur Auswertung wird die Mortalität, die prozentuale Hemmung der Ablage von fertilen Eiern (Blockierung der Embryogenese bzw. des Schlupfes) gegenüber unbehandelten Kontrollen ermittelt.

Die Verbindungen Nr. 1, 2 und 6 der Formel I gemäss Beispiel 1 zeigen 100 % Wirkung in obigem Test.

Beispiel 8: Wirkung gegen Zecken: Abtötungswirkung in verschiedenen Entwicklungsstadien

Als Testobjekte werden Larven (jeweils ca. 50), Nymphen (jeweils ca. 25) oder Imagines (jeweils ca. 10) der Zeckenarten Rhipicephalus bursa, Amblyomma hebraeum und Boophilus microplus verwendet. Die

Testtiere werden für kurze Zeit in wässrige Emulsionen der zu untersuchenden Substanzen mit einer Konzentration von 800 ppm getaucht. Die in Teströhrchen befindlichen Emulsionen werden dann mit Watte aufgenowen und die benetzten Testtiere in den so kontaminierten Röhrchen belassen. Die Auswertung (% - Mortalität) erfolgt für Larven nach 3 Tagen und für Nymphen und Imagines nach 14 Tagen.

Verbindungen der Formel I gemäss Beispiel 1 sind in diesem Test gut wirksam.

Beispiel 9: Ovizide Wirkung auf Cydia pomonella, Adoxophyes reticulana und Lobesia botrana

Abgelegte Eier der obigen Obstschädlinge, die nicht älter als 24 Stunden sind, werden dreimal für einige Sekunden in eine acetonisch-wässrige Lösung enthaltend 400 ppm des zu prüfenden Wirkstoffes eingetaucht. Nach dem Antrocknen der Test-Lösung werden die Eier in Petrischalen (5 cm Durchmesser) ausgelegt und bei einer Temperatur von etwa 26 °C und 55 % relativer Luftfeuchtigkeit belassen. Im Falle von Cydia pomonella (Apfelwickler) werden die behandelten Eigelege zwischen zwei Papierrundfiltern in der Petrischale deponiert. Im Falle von Adoxophyes reticulana (Fruchtschalenwickler) und Lobesia botrana (Traubenwickler) werden die Eigelege zwischen zwei Stoffrundfiltern unter dem Deckel der Petrischale deponiert, deren Unterteil mit einer normalen Lepidopteren-Diät ausgegossen ist. Nach 6 Tagen wird der prozentuale Schlupf von Larven aus den behandelten Eiern gegenüber unbehandelten Kontrollpflanzen bewertet.

Verbindungen der Formel I gemäss Beispiel 1 zeigen gute Wirkung in obigem Test.

Beispiel 10: Chemosterilisierungs-Wirkung auf Nilaparvata lugens

Der Test wird an wachsenden Pflanzen durchgeführt. Jeweils 4 Reispflanzen (Dicke des Stengels ca. 5 mm, Höhe ca. 20 cm) werden in Töpfe (Durchmesser von 8 cm) eingepflanzt.

Die Pflanzen werden auf einem Drehteller mit 5 ml einer wässrigen Emulsionszubereitung enthaltend 400 ppm des jeweiligen Wirkstoffes besprüht. Nach dem Antrocknen des Sprühbelages wird jede Pflanze mit 4 adulten Weibchen und 2 Männchen besiedelt. Um die Tiere am Entweichen zu hindern, wird über jeden besiedelten Topf ein durchsichtiger Kunststoff-Zylinder gestülpt und dieser mit einem Gaze-Deckel abgedeckt. Die Test-Tiere bleiben 6 Tage zur Eiablage auf den behandelten Pflanzen. Die überlebenden Tiere werden ausgezählt und dann entfernt.

Die Reispflanzen mit den abgelegten Eiern werden während 14 Tagen bei 26 °C und 60 % relativer Luftfeuchtigkeit inkubiert. Die in diesem Zeitraum geschlüpften jungen Zikaden werden ausgezählt. Aus dem Vergleich der Anzahl geschlüpfter Larven auf den behandleten Pflanzen zu den auf unbehandelten Kontrollpflanzen geschlüpften Tieren wird die prozentuale Reduktion der Nachkommenschaft (Chemosterilisierungs-Effekt) bestimmt.

Verbindungen der Formel I gemäss Beispiel 1 zeigen in obigem Test gute Wirkung.

Beispiel 11: Wirkung gegen Bemisia tabaci

a) Wirkstoffapplikation vor Besiedlung

Eingetopfte Baumwollpflanzen im Keimblattstadium werden bis zur Tropfnässe mit einer wässrigen Emulsionszubereitung des zu prüfenden Wirkstoffes (Konzentration 400 ppm) besprüht. Nach dem Antrocknen des Sprühbelages werden auf jeder Pflanze 40 Adulte Bemisia tabaci (Weisse Fliege) in Plastikzylindern auf den Pflanzen gehalten. Zur Auswertung wird die %-Mortalität der angesetzten Adulten drei Tage nach Besiedlung bestimmt, wonach die überlebenden Adulten entfernt werden. Eine zweite Auswertung erfolgt 24 Tage nach Besiedlung auf %-Mortalität der Nymphen, Puppen und Adulten der ersten Folgegeneration. Der Versuch wird in einem klimatisierten Raum bei 25 °C und einer relativen Luftfeuchtigkeit von etwa 50 bis 60 % durchgeführt.

b) Wirkstoffapplikation nach Besiedlung

Eingetopfte Baumwollpflanzen (unbehandelt) im Keimblattstadium werden wie unter a) angegeben mit Bemisia tabaci besiedelt (40 ungesexte Adulte pro Pflanze). Nach erfolgter Eiablage während drei Tagen werden alle vorhandenen Adulten entfernt. Zehn Tage nach der Besiedlung, d.h. zu einem Zeitpunkt, wo sich etw 2/3 der Nymphen im ersten Nymphen-stadium und 1/3 im zweiten Stadium befinden, werden die besiedelten Pflanzen bis zur Tropfnässe mit einer wässrigen Emulsions-Zubereitung des zu prüfenden Wirkstoffes (Konzentration 400 ppm) besprüht. Die Auswertung erfolgt 24 Tage nach der Besiedlung auf %-Mortalität der Nymphen, Puppen und Adulten. Der Versuch wird in einem klimatisierten Raum bei 25 °C und einer relativen Luftfeuchtigkeit von etwa 50 % bis 60 % durchgeführt.

Die Verbindungen der Formel I gemäss Beispiel 1 zeigen gute Wirkung in diesen Testen.

Beispiel 12: Wirkung gegen Vorratsschädlinge - Sitophilus orizae und Rhizopertha dominica

Es werden 100 g Weizenkörner in Plastikbecher von 100 ml Inhalt mit je 1 ml einer wässrigen Emulsionszubereitung des zu prüfenden Wirkstoffs gründlich vermischt, wobei die verwendete wässrige Emulsionszubereitung eine solche Wirkstoffkonzentration aufweist, dass sich, bezogen auf das Gewicht der Weizenkörner, eine End-Wirkstoffkonzentration von 100 ppm ergibt. Dann werden pro Becher (gefüllt mit 100 g behandelter Weizenkörner) 25 ungesexte Adulte von Sitophilus orizae (Reiskäfer) bzw. Rhizopertha dominica (Getreidekapuziner) angesetzt. Nach der Besiedlung werden die Ansätze bei 26 bis 28 °C und 60 bis 65 % relativer Luftfeuchtigkeit im Dunkeln gehalten. Die Auswertung erfolgt eine Woche nach Besiedlung auf %-Mortalität der angesetzten Adulten und 8 Wochen nach Besiedlung auf %-Reduktion der ersten Folgegeneration.

Die Verbindungen der Formel I gemäss Beispiel 1 zeigen gute Wirkung in diesem Test.

**Patentansprüche**

**Patentansprüche für folgende Vertragsstaaten : AT, BE, CH, DE, FR, GB, GR, IT, LI, NL**

**1.** Verbindung der Formel

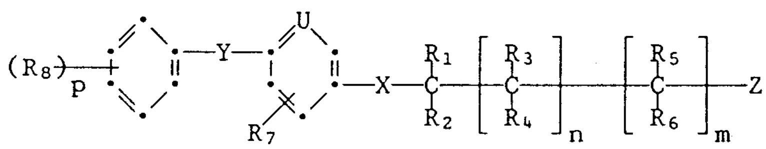

(I),

worin

| | |
|---|---|
| $R_1$, $R_2$, $R_3$, $R_4$, $R_5$ und $R_6$ | unabhängig voneinander Wasserstoff oder $C_1$-$C_4$-Alkyl oder, wenn n = 1, |
| $R_2$ und $R_4$ | zusammen einen der Reste |

$$-(CH_2)_3- \quad \text{oder} \quad -(CH_2)_4- \; ;$$

| | |
|---|---|
| $R_7$ | Wasserstoff, Halogen, Methyl, Aethyl, mit 1 bis 5 Halogenatomen substituiertes $C_1$-$C_2$-Alkyl, Methoxy, Aethoxy oder mit 1 bis 5 Halogenatomen substituiertes $C_1$-$C_2$-Alkoxy; |
| $R_8$ | Halogen, $C_1$-$C_3$-Alkyl, mit 1 bis 7 Halogenatomen substituiertes $C_1$-$C_3$-Alkyl, $C_1$-$C_3$-Alkoxy, mit 1 bis 7 Halogenatomen substituiertes $C_1$-$C_3$-Alkoxy oder Cyano; |
| U | eine Gruppierung -CH= oder -H= ; |
| X | -O- , -S- oder -N($R_9$)- ; |
| Y | -O- , -S- , -S(O)- , -S($O_2$)- , -$CH_2$- , -CO- oder -N($R_9$)- , wobei |
| $R_9$ | für Wasserstoff, $C_1$-$C_4$-Alkyl oder Alkylcarbonyl mit insgesamt 2 bis 4 C-Atomen steht; |
| n | eine Zahl 0 oder 1; |
| m | eine Zahl 0, 1 oder 2; |
| p | eine Zahl 1, 2 oder 3; und |
| Z | den Rest |

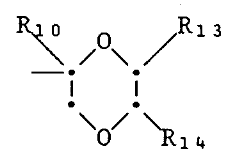

bedeuten, wobei

$R_{10}$ Wasserstoff, Methyl oder Aethyl; und
$R_{13}$ und $R_{14}$ unabhängig voneinander Wasserstoff, Halogen oder $C_1$-$C_3$-Alkyl; oder
$R_{13}$ und $R_{14}$ zusammen die Gruppe

$$-(CH_2)_4-$$

bedeuten.

**2.** Verbindung der Formel I gemäss Anspruch 1, dadurch gekennzeichnet, dass
$R_1$ und $R_3$ unabhängig voneinander Wasserstoff, Methyl oder Aethyl;
$R_2$, $R_4$, $R_5$ und $R_6$ Wasserstoff oder, wenn n = 1,
$R_2$ und $R_4$ zusammen einen der Reste

$$-(CH_2)_3- \text{ oder } -(CH_2)_4- \;;$$

$R_7$ Wasserstoff oder Halogen;
$R_8$ Methyl, Aethyl, Halogen oder mit 1 bis 5 Halogenatomen substituiertes $C_1$-$C_2$-Alkyl;
U eine Gruppierung -CH= oder -N= ;
X -O- , -S- oder -N($R_9$)- ;
Y -O- , -S- , -$CH_2$- oder -CO- ; wobei
$R_9$ für Wasserstoff, Methyl oder Acetyl steht;
n und m unabhängig voneinander eine Zahl 0 oder 1; und
p eine Zahl 1 oder 2; und
Z den Rest

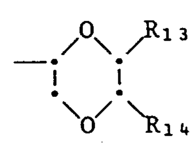

bedeuten, wobei
$R_{13}$ und $R_{14}$ unabhängig voneinander Wasserstoff oder Methyl bedeuten.

**3.** Verbindung der Formel I gemäss Anspruch 2, dadurch gekennzeichnet, dass
$R_1$ Wasserstoff oder Methyl;
$R_2$, $R_3$, $R_4$, $R_5$, $R_6$ und $R_7$ Wasserstoff;
$R_8$ Fluor, Chlor, Methyl oder Trifluormethyl;
U die Gruppierung -CH= ;
X -O- ;
Y -O- ;
n und m die Zahl 0;
p eine Zahl 1 oder 2; und
Z den Rest

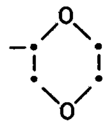

bedeuten.

**4.** Verbindung der Formel I gemäss Anspruch 1, dadurch gekennzeichnet, dass

| | |
|---|---|
| $R_1$ | Wasserstoff oder Methyl; |
| $R_2$, $R_3$, $R_4$, $R_5$, $R_6$ und $R_7$ | Wasserstoff |
| $R_8$ | Halogen oder Trifluormethyl; |
| U | die Gruppierung -CH=; |
| X | -O- oder -S-; |
| Y | -O- , -S- , $-CH_2-$ oder -CO-; |
| n und m | die Zahl 0; |
| p | eine Zahl 1 oder 2; und |
| Z | den Rest |

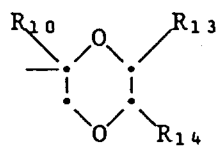

bedeuten, wobei

| | |
|---|---|
| $R_{10}$ | Wasserstoff oder Methyl; und |
| $R_{13}$ und $R_{14}$ | unabhängig voneinander Wasserstoff, Halogen oder $C_1$-$C_3$-Alkyl; oder |
| $R_{13}$ und $R_{14}$ | zusammen die Gruppe |

$$-(CH_2)_4-$$

bedeuten.

**5.** Verbindung der Formel I gemäss Anspruch 4, dadurch gekennzeichnet, dass

| | |
|---|---|
| $R_1$, $R_2$, $R_3$, $R_4$, $R_5$, $R_6$ und $R_7$ | Wasserstoff; |
| $R_8$ | Fluor, Chlor oder Trifluormethyl; |
| U | die Gruppierung -CH=; |
| X | -O- , |
| Y | -O- , -S- , $-CH_2-$ oder -CO- ; |
| n und m | die Zahl 0; |
| p | eine Zahl 1 oder 2; und |
| Z | den Rest |

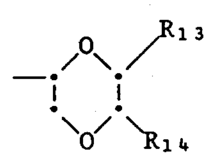

bedeuten, wobei

| | |
|---|---|
| $R_{13}$ und $R_{14}$ | unabhängig voneinander Wasserstoff, Halogen oder $C_1$-$C_3$-Alkyl; oder |
| $R_{13}$ und $R_{14}$ | zusammen die Gruppe |

$$-(CH_2)_4-$$

bedeuten.

6. Verbindung gemäss einem der Ansprüche 1 bis 5, dadurch gekennzeichnet, dass p = 1 ist.

7. Verbindung gemäss Anspruch 5 der Formel

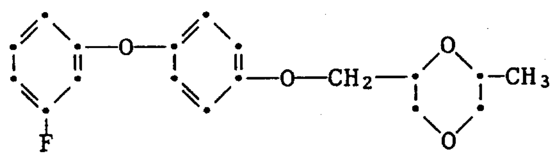

.

8. Verbindung gemäss Anspruch 6 der Formel

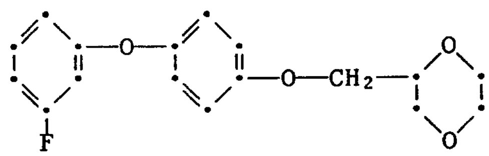

.

9. Verbindung gemäss Anspruch 6 der Formel

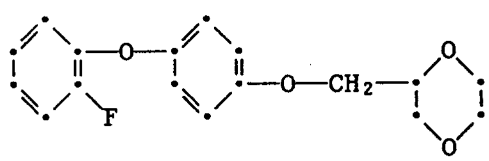

.

10. Verbindung gemäss Anspruch 6 der Formel

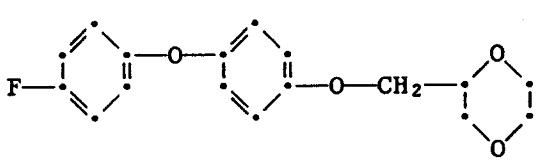

.

11. Verbindung gemäss Anspruch 5 der Formel

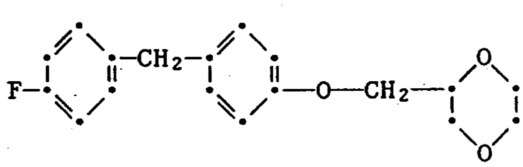

.

**12.** Verbindung gemäss Anspruch 5 der Formel

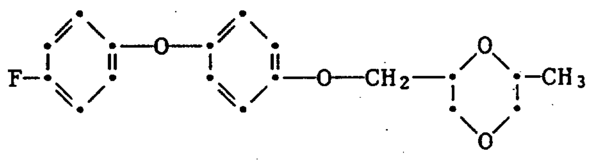

**13.** Verfahren zur Herstellung einer Verbindung der Formell I gemäss einem der Ansprüche 1 bis 12, dadurch gekennzeichnet, dass man eine Verbindung der Formel

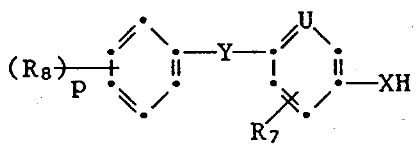

(II)

mit einer Verbindung der Formel

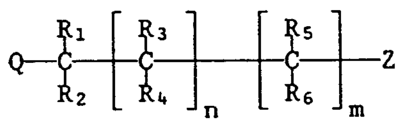

(III)

umsetzt, wobei in den Formeln II und III $R_1$ bis $R_8$, U, X, Y, n, m, p und Z die für die Formel I in die Ansprüchen 1 bis 6 angegebenen Bedeutungen haben und Q für eine Abgangsgruppe steht.

**14.** Schädlingsbekämpfungsmittel, welches als aktive Komponente eine Verbindung gemäss einem der Ansprüche 1 bis 12 zusammen mit geeigneten Trägern und/oder anderen Zuschlagstoffen enthält.

**15.** Verwendung einer Verbindung gemäss einem der Ansprüche 1 bis 12 zur Bekämpfung von Insekten und Vertretern der Ordnung Acarina.

**16.** Verwendung gemäss Anspruch 15 zur Bekämpfung von pflanzenschädigenden saugenden Insekten.

**17.** Verwendung gemäss Anspruch 16 zur Bekämpfung von Schildläusen.

**18.** Verwendung gemäss Anspruch 15 zur Bekämpfung larvaler Stadien pflanzenschädigender Insekten.

**19.** Verfahren zur Bekämpfung von Insekten und Vertretern der Ordnung Acarina, dadurch gekennzeichnet, dass man die Schädlinge, bzw. deren verschiedene Entwicklungsstadien oder deren Aufenthaltsort, mit einer pestizid wirksamen Menge einer Verbindung der Formel I gemäss einem der Ansprüche 1 bis 12 oder mit einem Mittel, enthaltend neben Zusatz- und Trägerstoffen eine pestizid wirksame Menge dieser Verbindung, in Kontakt bringt oder behandelt.

**Patentansprüche für folgenden Vertragsstaat : ES**

**1.** Verfahren zur Herstellung einer Verbindung der Formel

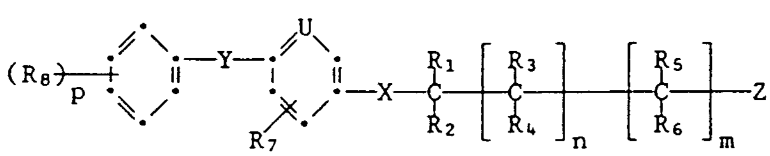

(I),

worin

$R_1$, $R_2$, $R_3$, $R_4$, $R_5$ und $R_6$ unabhängig voneinander Wasserstoff oder $C_1$-$C_4$-Alkyl oder, wenn n = 1,

$R_2$ und $R_4$ zusammen einen der Reste

$$-(CH_2)_3- \quad \text{oder} \quad -(CH_2)_4- \; ;$$

$R_7$ Wasserstoff, Halogen, Methyl, Aethyl, mit 1 bis 5 Halogenatomen substituiertes $C_1$-$C_2$-Alkyl, Methoxy, Aethoxy oder mit 1 bis 5 Halogenatomen substituiertes $C_1$-$C_2$-Alkoxy;

$R_8$ Halogen, $C_1$-$C_3$-Alkyl, mit 1 bis 7 Halogenatomen substituiertes $C_1$-$C_3$-Alkyl, $C_1$-$C_3$-Alkoxy, mit 1 bis 7 Halogenatomen substituiertes $C_1$-$C_3$-Alkoxy oder Cyano;

U eine Gruppierung -CH= oder -N= ;

X -O- , -S- oder -N($R_9$)- ;

Y -O- , -S- , -S(O)- , -S($O_2$)- , -$CH_2$- , -CO- oder -N($R_9$)- , wobei

$R_9$ für Wasserstoff, $C_1$-$C_4$-Alkyl oder Alkylcarbonyl mit insgesamt 2 bis 4 C-Atomen steht;

n eine Zahl 0 oder 1;

m eine Zahl 0, 1 oder 2;

p eine Zahl 1, 2 oder 3; und

Z den Rest

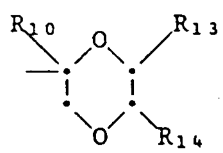

bedeuten, wobei

$R_{10}$ Wasserstoff, Methyl oder Aethyl; und

$R_{13}$ und $R_{14}$ unabhängig voneinander Wasserstoff, Halogen oder $C_1$-$C_3$-Alkyl; oder

$R_{13}$ und $R_{14}$ zusammen die Gruppe

$$-(CH_2)_4-$$

bedeuten,
dadurch gekennzeichnet, dass man eine Verbindung der Formel

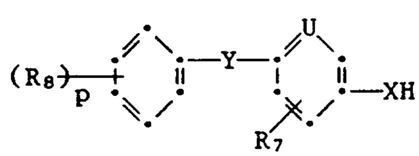

(II)

mit einer Verbindung der Formel

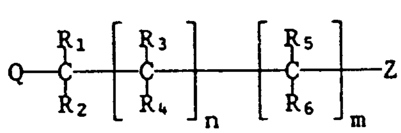

(III)

umsetzt, wobei in den Formeln II und III $R_1$ bis $R_8$ U, X, Y, n, m, p und Z die für die Formel I angegebenen Bedeutungen haben und Q für eine Abgangsgruppe steht.

**2.** Verfahren zur Herstellung einer Verbindung der Formel I gemäss Anspruch 1, dadurch gekennzeichnet, dass

$R_1$ und $R_3$ unabhängig voneinander Wasserstoff, Methyl oder Aethyl;
$R_2$, $R_4$, $R_5$ und $R_6$ Wasserstoff oder, wenn n = 1,
$R_2$ und $R_4$ zusammen einen der Reste

$-(CH_2)_3-$ oder $-(CH_2)_4-$ ;

$R_7$ Wasserstoff oder Halogen;
$R_8$ Methyl, Aethyl, Halogen oder mit 1 bis 5 Halogenatomen substituiertes $C_1$-$C_2$-Alkyl;
U eine Gruppierung -CH= oder -N= ;
X -O- , -S- oder -N($R_9$)- ;
Y -O- , -S- , -$CH_2$- oder -CO- ; wobei
$R_9$ für Wasserstoff, Methyl oder Acetyl steht;
n und m unabhängig voneinander eine Zahl 0 oder 1; und
p eine Zahl 1 oder 2; und
Z den Rest

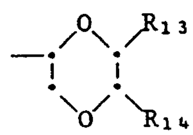

bedeuten, wobei
$R_{13}$ und $R_{14}$ unabhängig voneinander Wasserstoff oder Methyl bedeuten.

**3.** Verfahren zur Herstellung einer Verbindung der Formel I gemäss Anspruch 2, dadurch gekennzeichnet, dass

$R_1$ Wasserstoff oder Methyl;
$R_2$, $R_3$, $R_4$, $R_5$, $R_6$ und $R_7$ Wasserstoff;
$R_8$ Fluor, Chlor, Methyl oder Trifluormethyl;
U die Gruppierung -CH= ;
X -O- ;
Y -O- ;
n und m die Zahl 0;
p eine Zahl 1 oder 2; und
Z den Rest

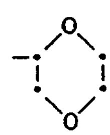

bedeuten.

**4.** Verfahren zur Herstellung einer Verbindung der Formel I gemäss Anspruch 1, dadurch gekennzeichnet, dass

$R_1$ Wasserstoff oder Methyl;
$R_2$, $R_3$, $R_4$, $R_5$, $R_6$ und $R_7$ Wasserstoff
$R_8$ Halogen oder Trifluormethyl;
U die Gruppierung -CH=;
X -O- oder -S-;
Y -O- , -S- , -$CH_2$- oder -CO-;
n und m die Zahl 0;

| | |
|---|---|
| p | eine Zahl 1 oder 2; und |
| Z | den Rest |

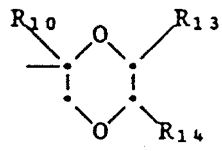

bedeuten, wobei

| | |
|---|---|
| $R_{10}$ | Wasserstoff oder Methyl; und |
| $R_{13}$ und $R_{14}$ | unabhängig voneinander Wasserstoff, Halogen oder $C_1$-$C_3$-Alkyl; oder |
| $R_{13}$ und $R_{14}$ | zusammen die Gruppe |

$$-(CH_2)_4-$$

bedeuten.

**5.** Verfahren zur Herstellung einer Verbindung der Formel I gemäss Anspruch 4 dadurch gekennzeichnet, dass

| | |
|---|---|
| $R_1$, $R_2$, $R_3$, $R_4$, $R_5$, $R_6$ und $R_7$ | Wasserstoff; |
| $R_6$ | Fluor, Chlor oder Trifluormethyl; |
| U | die Gruppierung -CH=; |
| X | -O- , |
| Y | -O- , -S- , -$CH_2$- oder -CO- ; |
| n und m | die Zahl 0; |
| p | eine Zahl 1 oder 2; und |
| Z | den Rest |

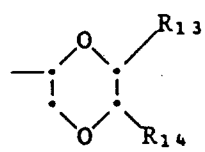

bedeuten, wobei

| | |
|---|---|
| $R_{13}$ und $R_{14}$ | unabhängig voneinander Wasserstoff, Halogen oder $C_1$-$C_3$-Alkyl; oder |
| $R_{13}$ und $R_{14}$ | zusammen die Gruppe |

$$-(CH_2)_4-$$

bedeuten.

**6.** Verfahren zur Herstellung einer Verbindung gemäss einem der Ansprüche 1 bis 5, dadurch gekennzeichnet, dass p = 1 ist.

**7.** Verfahren zur Herstellung einer Verbindung gemäss Anspruch 5 der Formel

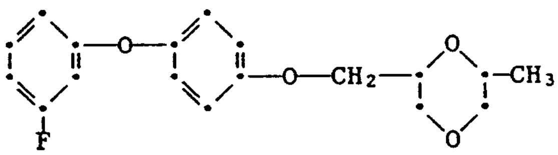

.

**8.** Verfahren zur Herstellung einer Verbindung gemäss Anspruch 6 der Formel

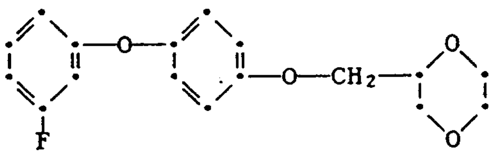

.

**9.** Verfahren zur Herstellung einer Verbindung gemäss Anspruch 6 der Formel

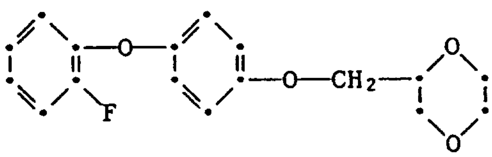

.

**10.** Verfahren zur Herstellung einer Verbindung gemäss Anspruch 6 der Formel

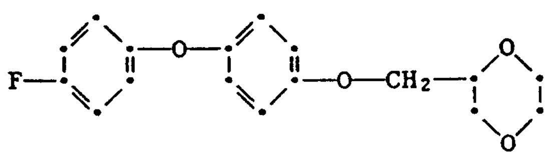

.

**11.** Verfahren zur Herstellung einer Verbindung gemäss Anspruch 5 der Formel

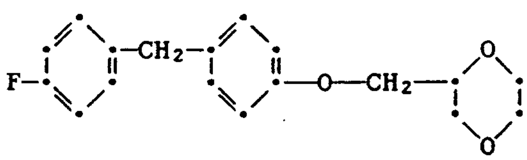

.

**12.** Verfahren zur einer Verbindung gemäss Anspruch 5 der Formel

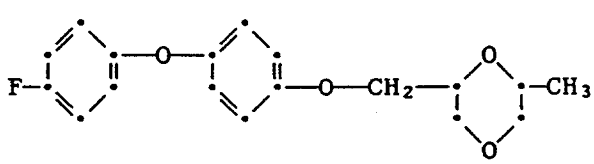

.

**13.** Schädlingsbekämpfungsmittel, welches als aktive Komponente eine Verbindung, erhältlich gemäss einem der Ansprüche 1 bis 12, zusammen mit geeigneten Trägern und/oder anderen Zuschlagstoffen enthält.

**14.** Verwendung einer Verbindung, erhältlich gemäss einem der Ansprüche 1 bis 12, zur Bekämpfung von Insekten und Vertretern der Ordnung Acarina.

**15.** Verwendung gemäss Anspruch 14 zur Bekämpfung von pflanzenschädigenden saugenden Insekten.

**16.** Verwendung gemäss Anspruch 15 zur Bekämpfung von Schildläusen.

**17.** Verwendung gemäss Anspruch 14 zur Bekämpfung larvaler Stadien pflanzenschädigender Insekten.

**18.** Verfahren zur Bekämpfung von Insekten und Vertretern der Ordnung Acarina, dadurch gekennzeichnet, dass man die Schädlinge, bzw. deren verschiedene Entwicklungsstadien oder deren Aufenthaltsort, mit einer pestizid wirksamen Menge einer Verbindung der Formel I, erhältlich gemäss einem der Ansprüche 1 bis 12, oder mit einem Mittel, enthaltend neben Zusatz- und Trägerstoffen eine pestizid wirksame Menge dieser Verbindung, in Kontakt bringt oder behandelt.

## Claims

**Claims for the following Contracting States : AT, BE, CH, DE, FR, GB, GR, IT, LI, NL**

**1.** A compound of formula

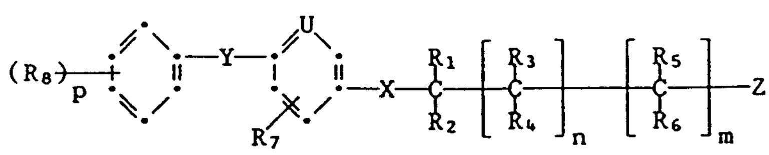

(I)

wherein

$R_1$, $R_2$, $R_3$, $R_4$, $R_5$ and $R_6$ each independently of the others represents hydrogen or $C_1$-$C_4$alkyl or, if n = 1, $R_2$ and $R_4$ together represent one of the radicals

$$-(CH_2)_3- \quad \text{or} \quad -(CH_2)_4-;$$

$R_7$ represents hydrogen, halogen, methyl, ethyl, $C_1$-$C_2$-alkyl substituted by 1 to 5 halogen atoms, methoxy, ethoxy, or $C_1$-$C_2$alkoxy substituted by 1 to 5 halogen atoms;
$R_8$ represents halogen, $C_1$-$C_3$alkyl, $C_1$-$C_3$alkyl substituted by 1 to 7 halogen atoms, $C_1$-$C_3$alkoxy, $C_1$-$C_3$alkoxy substituted by 1 to 7 halogen atoms, or cyano;
U represents a grouping -CH= or -N=;
X represents -O-, -S- or -N($R_9$)-;
Y represents -O-, -S-, -S(O)-, -S($O_2$)-, -$CH_2$-, -CO- or -N($R_9$)-, wherein
$R_9$ represents hydrogen, $C_1$-$C_4$alkyl, or alkylcarbonyl having a total of from 2 to 4 carbon atoms;
n is a number 0 or 1;
m is a number 0, 1 or 2;
p is a number 1, 2 or 3; and
Z represents the radical

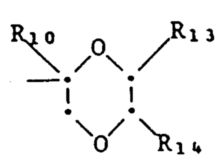

wherein
$R_{10}$ represents hydrogen, methyl or ethyl; and
$R_{13}$ and $R_{14}$ each independently of the other represents hydrogen, halogen or $C_1$-$C_3$alkyl; or
$R_{13}$ and $R_{14}$ together represent the group

$$-(CH_2)_4-.$$

2. A compound of formula I according to claim 1, wherein
$R_1$ and $R_3$ each independently of the other represents hydrogen, methyl or ethyl;
$R_2$, $R_4$, $R_5$ and $R_6$ represent hydrogen or, if n = 1,
$R_2$ and $R_4$ together represent one of the radicals

$$-(CH_2)_3- \quad \text{or} \quad -(CH_2)_4-;$$

$R_7$ represents hydrogen or halogen;
$R_8$ represents methyl, ethyl, halogen, or $C_1$-$C_2$alkyl substituted by 1 to 5 halogen atoms;
U represents a grouping -CH= or -N=;
X represents -O-, -S- or -N($R_9$)-;
Y represents -O-, -S-, -$CH_2$- or -CO-, wherein
$R_9$ represents hydrogen, methyl or acetyl;
n and m each independently of the other is a number 0 or 1; and
p is a number 1 or 2; and
Z represents the radical

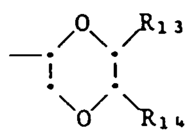

wherein
$R_{13}$ and $R_{14}$ each independently of the other represents hydrogen or methyl.

3. A compound of formula I according to claim 2, wherein $R_1$ represents hydrogen or methyl;
$R_2$, $R_3$, $R_4$, $R_5$, $R_6$ and $R_7$ represent hydrogen;
$R_8$ represents fluorine, chlorine, methyl or trifluoromethyl;
U represents the grouping -CH=;
X represents -O-;
Y represents -O-;
n and m are the number 0;
p is a number 1 or 2; and
Z represents the radical

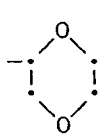

.

4. A compound of formula I according to claim 1, wherein
$R_1$ represents hydrogen or methyl;
$R_2$, $R_3$, $R_4$, $R_5$, $R_6$ and $R_7$ represent hydrogen;

$R_8$ represents halogen or trifluoromethyl;
U represents the grouping -CH=;
X represents -O- or -S-;
Y represents -O-, -S-, $-CH_2-$ or -CO-;
n and m are the number 0;
p is a number 1 or 2; and
Z represents the radical wherein

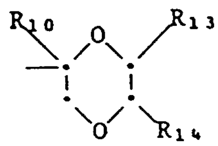

$R_{10}$ represents hydrogen or methyl; and
$R_{13}$ and $R_{14}$ each independently of the other represents hydrogen, halogen or $C_1$-$C_3$alkyl; or
$R_{13}$ and $R_{14}$ together represent the group

$$-(CH_2)_4-.$$

**5.** A compound of formula I according to claim 4, wherein
$R_1$, $R_2$, $R_3$, $R_4$, $R_5$, $R_6$ and $R_7$ represent hydrogen;
$R_8$ represents fluorine, chlorine or trifluoromethyl;
U represents the grouping -CH=;
X represents -O-;
Y represents -O-, -S-, $-CH_2-$ or -CO-;
n and m are the number 0;
p is a number 1 or 2; and
Z represents the radical

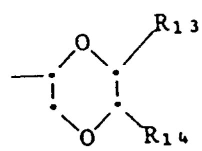

wherein
$R_{13}$ and $R_{14}$ each independently of the other represents hydrogen, halogen or $C_1$-$C_3$alkyl; or
$R_{13}$ and $R_{14}$ together represent the group

$$-(CH_2)_4-.$$

**6.** A compound according to any one of claims 1 to 5, wherein p = 1.

**7.** A compound according to claim 5 of formula

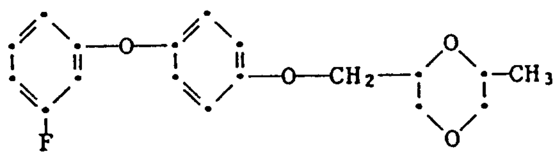
.

**8.** A compound according to claim 6 of formula

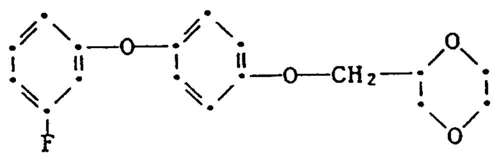
.

**9.** A compound according to claim 6 of formula

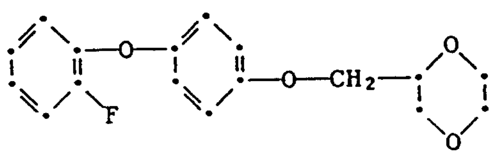
.

**10.** A compound according to claim 6 of formula

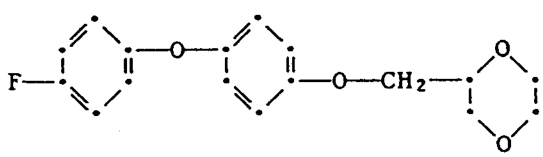
.

**11.** A compound according to claim 5 of formula

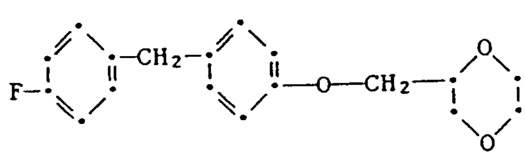
.

**12.** A compound according to claim 5 of formula

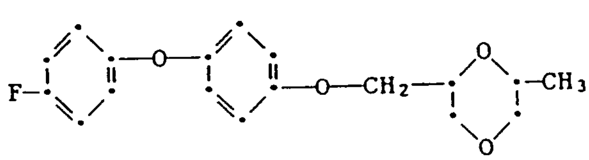
.

**13.** A process for the preparation of a compound of formula I according to any one of claims 1 to 12, which comprises reacting a compound of formula

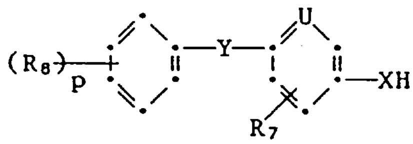

(II)

with a compound of formula

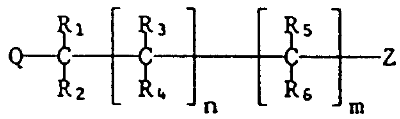

(III),

in which formulae II and III $R_1$ to $R_8$, U, X, Y, n, m, p and Z have the meanings given for formula I in claims 1 to 6, and Q represents a leaving group.

**14.** A pesticidal composition which comprises as active ingredient a compound according to any one of claims 1 to 12 together with suitable carriers and/or other adjuvants.

**15.** The use of a compound according to any one of claims 1 to 12 for controlling insects and representatives of the order Acarina.

**16.** The use according to claim 15 for controlling plant-destructive sucking insects.

**17.** The use according to claim 16 for controlling scale insects.

**18.** The use according to claim 15 for controlling larval stages of plant-destructive insects.

**19.** A method of controlling insects and representatives of the order Acarina, wherein the pests, or the various stages of development thereof, or their locus, is/are brought into contact or treated with a pesticidally effective amount of a compound of formula I according to any one of claims 1 to 12 or with a composition comprising a pesticidally effective amount of that compound together with adjuvants and carriers.

**Claims for the following Contracting State : ES**

**1.** A process for the preparation of a compound of formula

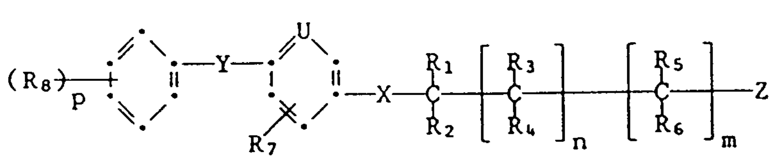

(I)

wherein
$R_1$, $R_2$, $R_3$, $R_4$, $R_5$ and $R_6$ each independently of the others represents hydrogen or $C_1$-$C_4$alkyl or, if n = 1,
$R_2$ and $R_4$ together represent one of the radicals

$$-(CH_2)_3- \quad \text{or} \quad -(CH_2)_4-;$$

$R_7$ represents hydrogen, halogen, methyl, ethyl, $C_1$-$C_2$-alkyl substituted by 1 to 5 halogen atoms, methoxy, ethoxy, or $C_1$-$C_2$alkoxy substituted by 1 to 5 halogen atoms;
$R_8$ represents halogen, $C_1$-$C_3$alkyl, $C_1$-$C_3$alkyl substituted by 1 to 7 halogen atoms, $C_1$-$C_3$alkoxy, $C_1$-$C_3$alkoxy substituted by 1 to 7 halogen atoms, or cyano;
U represents a grouping -CH= or -N=;
X represents -O-, -S- or -N($R_9$)-;
Y represents -O-, -S-, -S(O)-, -S($O_2$)-, -$CH_2$-, -CO- or -N($R_9$)-, wherein
$R_9$ represents hydrogen, $C_1$-$C_4$alkyl, or alkylcarbonyl having a total of from 2 to 4 carbon atoms;
n is a number 0 or 1;
m is a number 0, 1 or 2;
p is a number 1, 2 or 3; and
Z represents the radical

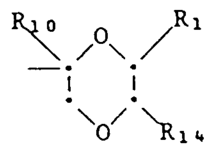

wherein
$R_{10}$ represents hydrogen, methyl or ethyl; and
$R_{13}$ and $R_{14}$ each independently of the other represents hydrogen, halogen or $C_1$-$C_3$alkyl; or
$R_{13}$ and $R_{14}$ together represent the group

$$-(CH_2)_4-,$$

which process comprises reacting a compound of formula

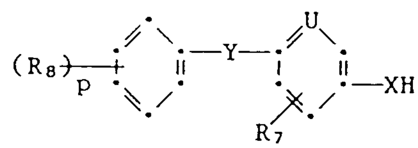

(II)

with a compound of formula

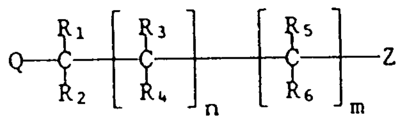

(III),

in which formulae II and III $R_1$ to $R_8$, U, X, Y, n, m, p and Z have the meanings given for formula I, and Q represents a leaving group.

**2.** A process for the preparation of a compound of formula I according to claim 1, wherein
$R_1$ and $R_3$ each independently of the other represents hydrogen, methyl or ethyl;
$R_2$, $R_4$, $R_5$ and $R_6$ represent hydrogen or, if n = 1,
$R_2$ and $R_4$ together represent one of the radicals

$$-(CH_2)_3- \quad \text{or} \quad -(CH_2)_4-;$$

$R_7$ represents hydrogen or halogen;
$R_8$ represents methyl, ethyl, halogen, or $C_1$-$C_2$alkyl substituted by 1 to 5 halogen atoms;
U represents a grouping -CH= or -N=;

X represents -O-, -S- or $-N(R_9)-$;
Y represents -O-, -S-, $-CH_2-$ or -CO-, wherein
$R_9$ represents hydrogen, methyl or acetyl;
n and m each independently of the other is a number 0 or 1; and
p is a number 1 or 2; and
Z represents the radical

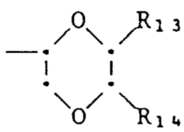

wherein
$R_{13}$ and $R_{14}$ each independently of the other represents hydrogen or methyl.

**3.** A process for the preparation of a compound of formula I according to claim 2, wherein
$R_1$ represents hydrogen or methyl;
$R_2$, $R_3$, $R_4$, $R_5$, $R_6$ and $R_7$ represent hydrogen;
$R_8$ represents fluorine, chlorine, methyl or trifluoromethyl;
U represents the grouping -CH=;
X represents -O-;
Y represents -O-;
n and m are the number 0;
p is a number 1 or 2; and
Z represents the radical

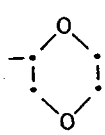 .

**4.** A process for the preparation of a compound of formula I according to claim 1, wherein
$R_1$ represents hydrogen or methyl;
$R_2$, $R_3$, $R_4$, $R_5$, $R_6$ and $R_7$ represent hydrogen;
$R_8$ represents halogen or trifluoromethyl;
U represents the grouping -CH=;
X represents -O- or -S-;
Y represents -O-, -S-, $-CH_2-$ or -CO-;
n and m are the number 0;
p is a number 1 or 2; and
Z represents the radical

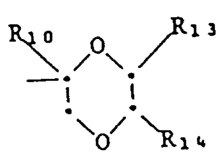

wherein
$R_{10}$ represents hydrogen or methyl; and
$R_{13}$ and $R_{14}$ each independently of the other represents hydrogen, halogen or $C_1$-$C_3$alkyl; or
$R_{13}$ and $R_{14}$ together represent the group

$-(CH_2)_4-$.

5. A process for the preparation of a compound of formula I according to claim 4, wherein
$R_1$, $R_2$, $R_3$, $R_4$, $R_5$, $R_6$ and $R_7$ represent hydrogen;
$R_8$ represents fluorine, chlorine or trifluoromethyl;
U represents the grouping -CH=;
X represents -O-;
Y represents -O-, -S-, $-CH_2-$ or -CO-;
n and m are the number 0;
p is a number 1 or 2; and
Z represents the radical

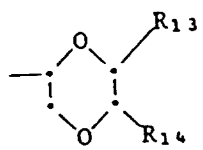

wherein
$R_{13}$ and $R_{14}$ each independently of the other represents hydrogen, halogen or $C_1$-$C_3$alkyl; or
$R_{13}$ and $R_{14}$ together represent the group

$-(CH_2)_4-$.

6. A process for the preparation of a compound according to any one of claims 1 to 5, wherein p = 1.

7. A process for the preparation of a compound according to claim 5 of formula

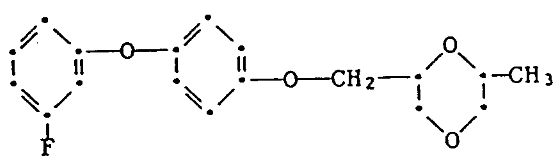

8. A process for the preparation of a compound according to claim 6 of formula

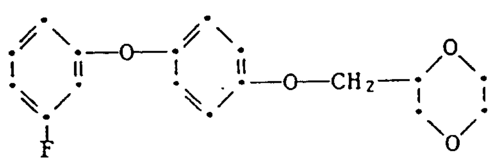

**9.** A process for the preparation of a compound according to claim 6 of formula

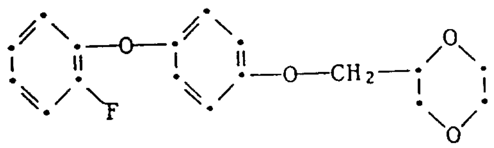

.

**10.** A process for the preparation of a compound according to claim 6 of formula

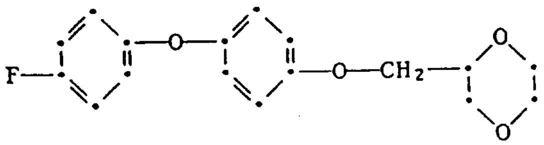

.

**11.** A process for the preparation of a compound according to claim 5 of formula

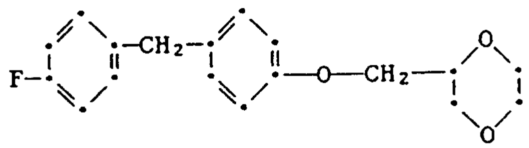

.

**12.** A process for the preparation of a compound according to claim 5 of formula

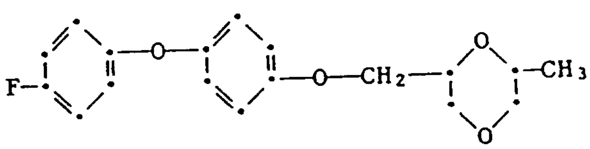

.

**13.** A pesticidal composition which comprises as active ingredient a compound obtainable according to any one of claims 1 to 12, together with suitable carriers and/or other adjuvants.

**14.** The use of a compound obtainable according to any one of claims 1 to 12 for controlling insects and representatives of the order Acarina.

**15.** The use according to claim 14 for controlling plant-destructive sucking insects.

**16.** The use according to claim 15 for controlling scale insects.

**17.** The use according to claim 14 for controlling larval stages of plant-destructive insects.

**18.** A method of controlling insects and representatives of the order Acarina, wherein the pests, or the various stages of development thereof, or their locus, is/are brought into contact or treated with a pesticidally effective amount of a compound of formula I obtainable according to any one of claims 1 to 12, or with a composition comprising a pesticidally effective amount of that compound together with adjuvants and carriers.

## Revendications

**Revendications pour les Etats contractants suivants : AT, BE, CH, DE, FR, GB, GR, IT, LI, NL**

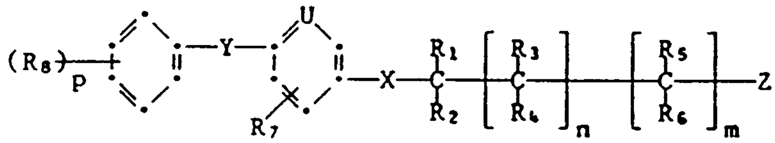

(I),

**1.** Composé de formule dans laquelle

$R_1$, $R_2$, $R_3$, $R_4$, $R_5$ et $R_6$ représentent, indépendamment les uns des autres, un atome d'hydrogène ou un groupe alkyle en $C_1$-$C_4$, ou, lorsque n = 1,

$R_2$ et $R_4$ représentent ensemble l'un des radicaux

$$-(CH_2)_3- \quad et \quad -(CH_2)_4-;$$

$R_7$ représente un atome d'hydrogène ou d'halogène ou le groupe méthyle, éthyle, un groupe alkyle en $C_1$-$C_2$ substitué par 1 à 5 atomes d'halogène, le groupe méthoxy, éthoxy ou un groupe alcoxy en $C_1$-$C_2$ substitué par 1 à 5 atomes d'halogène;

$R_8$ représente un atome d'halogène ou un groupe alkyle en $C_1$-$C_3$, alkyle en $C_1$-$C_3$ substitué par 1 à 7 atomes d'halogène, un groupe alcoxy en $C_1$-$C_3$, alcoxy en $C_1$-$C_3$ substitué par l à 7 atomes d'alogène ou le groupe cyano;

U représente le groupe -CH= ou -N=;

X représente -O-, -S- ou -N($R_9$) -;

Y représente -O-, -S-, -S(O), -S($O_2$)-, -$CH_2$-, -CO- ou -N($R_9$)-, $R_9$ représentant un atome d'hydrogène ou un groupe alkyle en $C_1$-$C_4$ ou alkylcarbonyle ayant au total de 2 à 4 atomes de carbone;

n est le nombre 0 ou 1;

m est le nombre 0, 1 ou 2;

p est le nombre 1, 2 ou 3; et

Z représente le radical

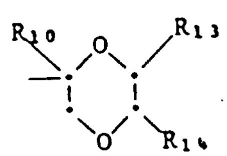

$R_{10}$ représentant un atome d'hydrogène ou le groupe méthyle ou éthyle; et

$R_{13}$ et $R_{14}$ représentant, indépendamment l'un de l'autre, un atome d'hydrogène ou d'halogène ou un groupe alkyle en $C_1$-$C_3$; ou

$R_{13}$ et $R_{14}$ formant ensemble le groupe

$$-(CH_2)_4-.$$

**2.** Composé de formule I selon la revendication 1, caractérisé en ce que

$R_1$ et $R_3$ représentent, indépendamment l'un de l'autre, un atome d'hydrogène ou le groupe méthyle ou éthyle;

$R_2$, $R_4$, $R_5$ ou $R_6$ représentent un atome d'hydrogène ou, lorsque n = 1,
$R_2$ et $R_4$ représentent ensemble l'un des radicaux

$$-(CH_2)_3- \quad \text{et} \quad -(CH_2)_4-;$$

$R_7$ représente un atome d'hydrogène ou d'halogène;
$R_8$ représente un atome d'halogène ou le groupe méthyle, éthyle ou un groupe alkyle en $C_1$-$C_2$ substitué par 1 à 5 atomes d'halogène;
U représente le groupement -CH= ou -N=;
X représente -O-, -S- ou -N($R_9$) - ;
Y représente -O-, -S-, -$CH_2$- ou -CO-;
$R_9$ représentant un atome d'hydrogène ou le groupe méthyle ou acétyle;
n et m représentent, indépendamment l'un de l'autre, le nombre 0 ou 1; et
p représente le nombre 1 ou 2; et
Z représente le radical

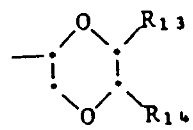

$R_{13}$ et $R_{14}$ représentant, indépendamment l'un de l'autre, un atome d'hydrogène ou le groupe méthyle.

**3.** Composé de formule I selon la revendication 2, caractérisé en ce que
$R_1$ représente un atome d'hydrogène ou le groupe méthyle;
$R_2$, $R_3$, $R_4$, $R_5$, $R_6$ et $R_7$ représentent des atomes d'hydrogène;
$R_8$ représente un atome de fluor ou de chlore ou le groupe méthyle ou trifluorométhyle;
U représente le groupement -CH=;
X représente -O-;
Y représente -O-;
n et m représentent le nombre 0;
p représente le nombre 1 ou 2; et
Z représente le radical

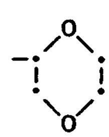

**4.** Composé de formule I selon la revendication 1, caractérisé en ce que
$R_1$ représente un atome d'hydrogène ou le groupe méthyle;
$R_2$, $R_3$, $R_4$, $R_5$, $R_6$ et $R_7$ représentent des atomes d'hydrogène;
$R_8$ représente un atome d'halogène ou le groupe trifluorométhyle;
U représente le groupement -CH=;
X représente -O- ou -S-;
Y représente -O-, -S-, -$CH_2$- ou -CO-;
n et m représentent le nombre 0;
p représente le nombre 1 ou 2; et
Z représente le radical

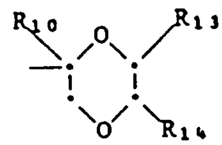

$R_{10}$ représentant un atome d'hydrogène ou le groupe méthyle; et

$R_{13}$ et $R_{14}$ représentant, indépendamment l'un de l'autre, un atome d'hydrogène ou d'halogène ou un groupe alkyle en $C_1$-$C_3$; ou

$R_{13}$ et $R_{14}$ représentant ensemble le groupe

$$-(CH_2)_4-.$$

**5.** Composé de formule I selon la revendication 4, caractérisé en ce que

$R_1$, $R_2$, $R_3$, $R_4$, $R_5$, $R_6$ et $R_7$ représentent des atomes d'hydrogène;

$R_8$ représente un atome de fluor ou de chlore ou le groupe trifluorométhyle;

U représente le groupement -CH=;

X représente -O-;

Y représente -O-, -S-, $-CH_2-$ ou -CO-;

n et m représentent le nombre 0;

p représente le nombre 1 ou 2; et

Z représente le radical

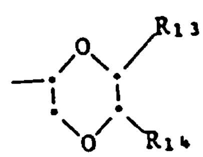

$R_{13}$ et $R_{14}$ représentant, indépendamment l'un de l'autre, un atome d'hydrogène ou d'halogène ou un groupe alkyle en $C_1$-$C_3$; ou

$R_{13}$ et $R_{14}$ représentant ensemble le groupe

$$-(CH_2)_4-.$$

**6.** Composé selon l'une des revendications 1 à 5, caractérisé en ce que p = 1.

**7.** Composé selon la revendication 5, de formule

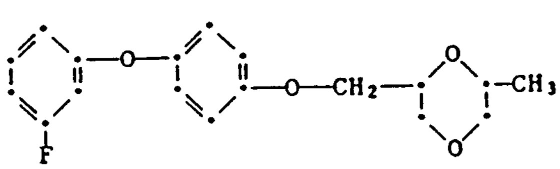

**8.** Composé selon la revendication 6, de formule

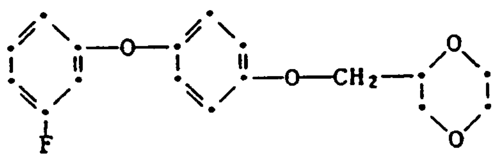

**9.** Composé selon la revendication 6, de formule

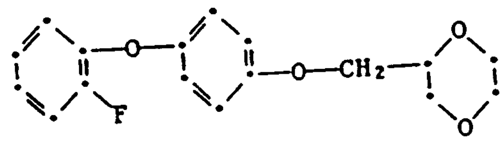

**10.** Composé selon la revendication 6, de formule

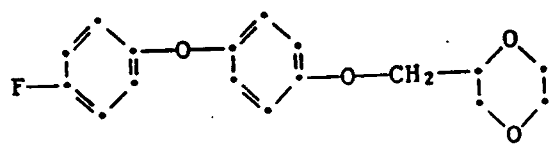

**11.** Composé selon la revendication 5, de formule

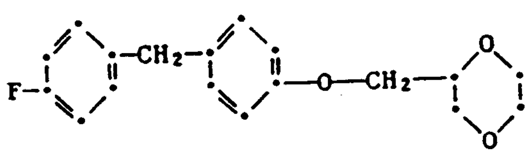

**12.** Composé selon la revendication 5, de formule

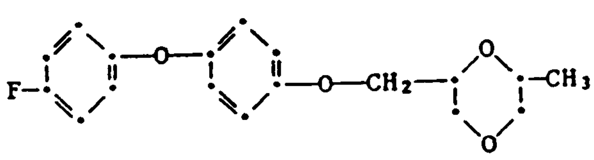

**13.** Procédé pour la préparation d'un composé de formule I selon l'une des revendications 1 à 12, caractérisé en ce que l'on fait réagir un composé de formule

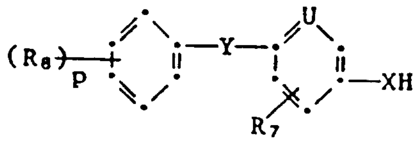

(II)

avec un composé de formule

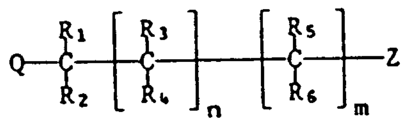

(III)

dans les formules II et III $R_1$ à $R_8$, U, X, Y, n, m, p et Z ayant les significations données pour la formule I dans les revendications 1 à 6, et Q représentant un groupe partant.

**14.** Produit de lutte contre des nuisibles, qui contient en tant que composant actif un composé selon l'une des revendications 1 à 12, conjointement avec des supports et/ou autres additifs appropriés.

**15.** Utilisation d'un composé selon l'une des revendications 1 à 12, pour la lutte contre des insectes et des représentants de la sous-classe des acariens.

**16.** Utilisation selon la revendication 15, pour la lutte contre des insectes suceurs nuisibles pour les plantes.

**17.** Utilisation selon la revendication 16, pour la lutte contre des cochenilles.

**18.** Utilisation selon la revendication 15, pour la lutte contre des stades larvaires d'insectes nuisibles pour les plantes.

**19.** Procédé pour la lutte contre des insectes et des représentants de la sous-classe des acariens, caractérisé en ce que l'on met en contact ou traite les nuisibles, ou leurs divers stades de développement ou leur habitat, par une quantité à activité pesticide d'un composé de formule I selon l'une des revendications 1 à 12 ou par un produit contenant, en plus d'additifs et de matériaux de support, une quantité à activité pesticide de ce composé.

**Revendications pour l'Etat contractant suivant : ES**

**1.** Procédé pour la préparation d'un composé de formule

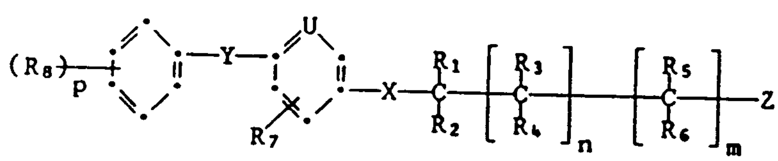

(I),

dans laquelle

| | |
|---|---|
| $R_1$, $R_2$, $R_3$, $R_4$, $R_5$ et $R_6$ | représentent, indépendamment les uns des autres, un atome d'hydrogène ou un groupe alkyle en $C_1$-$C_4$, ou, lorsque n = 1, |
| $R_2$ et $R_4$ | représentent ensemble l'un des radicaux |

$$-(CH_2)_3- \quad \text{et} \quad -(CH_2)_4-;$$

| | |
|---|---|
| $R_7$ | représente un atome d'hydrogène ou d'halogène ou le groupe méthyle, éthyle, un groupe alkyle en $C_1$-$C_2$ substitué par 1 à 5 atomes d'halogène, le groupe méthoxy, éthoxy ou un groupe alcoxy en $C_1$-$C_2$ substitué par 1 à 5 atomes d'halogène; |
| $R_8$ | représente un atome d'halogène ou un groupe alkyle en $C_1$-$C_3$, alkyle en $C_1$-$C_3$ substitué par 1 à 7 atomes d'halogène, un groupe alcoxy en $C_1$-$C_3$, alcoxy en $C_1$-$C_3$ substitué par 1 à 7 atomes d'alogène ou le groupe cyano; |

U représente le groupe -CH= ou -N=;
X représente -O-, -S- ou $-N(R_9)-$;
Y représente -O-, -S-, -S(O), $-S(O_2)-$, $-CH_2-$, -CO- ou $-N(R_9)-$, $R_9$ représentant un atome d'hydrogène ou un groupe alkyle en $C_1$-$C_4$ ou alkylcarbonyle ayant au total de 2 à 4 atomes de carbone;
n est le nombre 0 ou 1;
m est le nombre 0, 1 ou 2;
p est le nombre 1, 2 ou 3; et
Z représente le radical

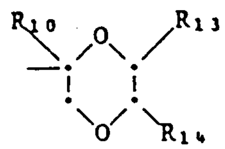

$R_{10}$ représentant un atome d'hydrogène ou le groupe méthyle ou éthyle; et
$R_{13}$ et $R_{14}$ représentant, indépendamment l'un de l'autre, un atome d'hydrogène ou d'halogène ou un groupe alkyle en $C_1$-$C_3$; ou
$R_{13}$ et $R_{14}$ formant ensemble le groupe

$$-(CH_2)_4-,$$

caractérisé en ce que l'on fait réagir un composé de formule

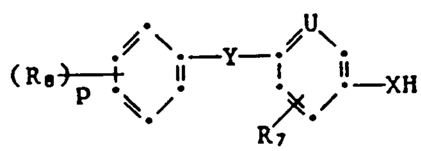 (II)

avec un composé de formule

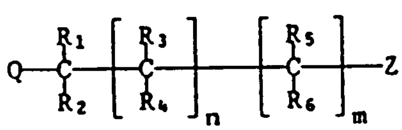 (III)

dans les formules II et III $R_1$ à $R_8$, U, X, Y, n, m, p et Z ayant les significations données pour la formule I et Q représentant un groupe partant.

**2.** Procédé pour la préparation d'un composé de formule I selon la revendication 1, caractérisé en ce que
$R_1$ et $R_3$ représentent, indépendamment l'un de l'autre, un atome d'hydrogène ou le groupe méthyle ou éthyle;
$R_2$, $R_4$, $R_5$ ou $R_6$ représentent un atome d'hydrogène ou, lorsque n = 1,
$R_2$ et $R_4$ représentent ensemble l'un des radicaux

$$-(CH_2)_3- \quad et \quad (CH_2)_4;$$

$R_7$ représente un atome d'hydrogène ou d'halogène;
$R_8$ représente un atome d'halogène ou le groupe méthyle, éthyle ou un groupe alkyle en $C_1$-$C_2$ substitué par 1 à 5 atomes d'halogène;
U représente le groupement -CH= ou -N-;

| | |
|---|---|
| X | représente -O-, -S- ou -N($R_9$) -; |
| Y | représente -O-, -S-, -$CH_2$- ou -CO-; |
| $R_9$ | représentant un atome d'hydrogène ou le groupe méthyle ou acétyle; |
| n et m | représentent, indépendamment l'un de l'autre, le nombre 0 ou 1; et |
| p | représente le nombre 1 ou 2, et |
| Z | représente le radical |

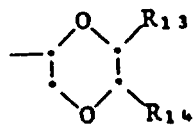

| | |
|---|---|
| $R_{13}$ et $R_{14}$ | représentant, indépendarnent l'un de l'autre, un atome d'hydrogène ou le groupe méthyle. |

**3.** Procédé pour la préparation d'un composé de formule I selon la revendication 2, caractérisé en ce que

| | |
|---|---|
| $R_1$ | représente un atome d'hydrogène ou le groupe méthyle; |
| $R_2$, $R_3$, $R_4$, $R_5$, $R_6$ et $R_7$ | représentent des atomes d'hydrogène; |
| $R_8$ | représente un atome de fluor ou de chlore ou le groupe méthyle ou trifluorométhyle; |
| U | représente le groupement -CH=; |
| X | représente -O-; |
| Y | représente -O-; |
| n et m | représentent le nombre 0; |
| p | représente le nombre 1 ou 2; et |
| Z | représente le radical |

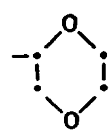

**4.** Procédé pour la préparation d'un composé de formule I selon la revendication 1, caractérisé en ce que

| | |
|---|---|
| $R_1$ | représente un atome d'hydrogène ou le groupe méthyle; |
| $R_2$, $R_3$, $R_4$, $R_5$, $R_6$ et $R_7$ | représentent des atomes d'hydrogène; |
| $R_8$ | représente un atome d'halogène ou le groupe trifluorométhyle; |
| U | représente le groupement -CH=; |
| X | représente -O- ou -S-; |
| Y | représente -O-, -S-, -$CH_2$- ou -CO-; |
| n et m | représentent le nombre 0; |
| p | représente le nombre 1 ou 2; et |
| Z | représente le radical |

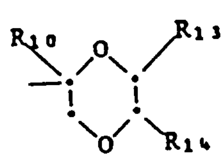

| | |
|---|---|
| $R_{10}$ | représentant un atome d'hydrogène ou le groupe méthyle; et |
| $R_{13}$ et $R_{14}$ | représentant, indépendamment l'un de l'autre, un atome d'hydrogène ou d'halogène ou un groupe alkyle en $C_1$-$C_3$; ou |

$R_{13}$ et $R_{14}$ représentant ensemble le groupe

$-(-CH_2-)_4-$.

5. Procédé pour la préparation d'un composé de formule I selon la revendication 4, caractérisé en ce que

| | |
|---|---|
| $R_1$, $R_2$, $R_3$, $R_4$, $R_5$, $R_6$ et $R_7$ | représentent des atomes d'hydrogène; |
| $R_8$ | représente un atome de fluor ou de chlore ou le groupe trifluorométhyle; |
| U | représente le groupement -CH=; |
| X | représente -O-; |
| Y | représente -O-, -S-, $-CH_2-$ ou -CO-; |
| n et m | représentent le nombre 0; |
| p | représente le nombre 1 ou 2; et |
| Z | représente le radical |

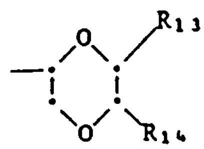

| | |
|---|---|
| $R_{13}$ et $R_{14}$ | représentant, indépendamment l'un de l'autre, un atome d'hydrogène ou d'halogène ou un groupe alkyle en $C_1$-$C_3$; ou |
| $R_{13}$ et $R_{14}$ | représentant ensemble le groupe |

$-(-CH_2-)_4-$.

6. Procédé pour la préparation d'un composé selon l'une des revendications 1 à 5, caractérisé en ce que p = 1.

7. Procédé pour la préparation d'un composé selon la revendication 5, de formule

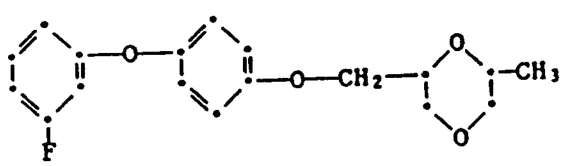

8. Procédé pour la préparation d'un composé selon la revendication 6, de formule

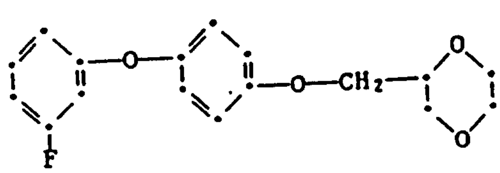

**9.** Procédé pour la préparation d'un composé selon la revendication 6, de formule

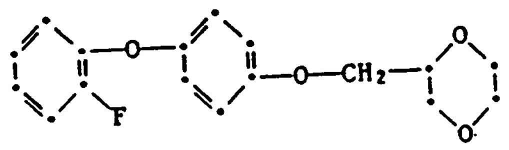

**10.** Procédé pour la préparation d'un composé selon la revendication 6, de formule

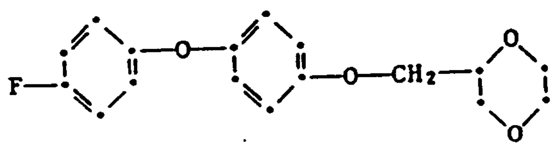

**11.** Procédé pour la préparation d'un composé selon la revendication 5, de formule

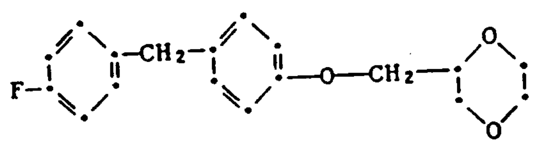

**12.** Procédé pour la préparation d'un composé selon la revendication 5, de formule

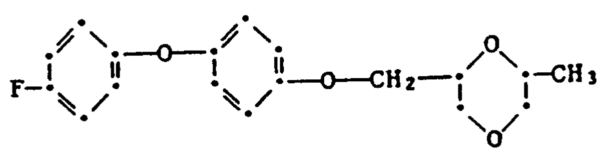

**13.** Produit de lutte contre des nuisibles, qui contient en tant que composant actif un composé préparable selon l'une des revendications 1 à 12, conjointement avec des supports et/ou autres additifs appropriés.

**14.** Utilisation d'un composé préparable selon l'une des revendications 1 à 12, pour la lutte contre des insectes et des représentants de la sous-classe des acariens.

**15.** Utilisation selon la revendication 14, pour la lutte contre des insectes suceurs nuisibles pour les plantes.

**16.** Utilisation selon la revendication 15, pour la lutte contre des cochenilles.

**17.** Utilisation selon la revendication 16, pour la lutte contre des stades larvaires d'insectes nuisibles pour les plantes.

**18.** Procédé pour la lutte contre des insectes et des représentants de la sous-classe des acariens, caractérisé en ce que l'on met en contact ou traite les nuisibles, ou leurs divers stades de développement ou leur habitat, par une quantité à activité pesticide d'un composé de formule I préparable selon l'une des revendications 1 à 12 ou par un produit contenant, en plus d'additifs et de

matériaux de support, une quantité à activité pesticide de ce composé.